(19) **Europäisches Patentamt**

**European Patent Office**

**Office européen des brevets**

(11) **EP 1 288 193 B1**

(12) **EUROPÄISCHE PATENTSCHRIFT**

(45) Veröffentlichungstag und Bekanntmachung des Hinweises auf die Patenterteilung:
**14.09.2005 Patentblatt 2005/37**

(51) Int Cl.⁷: $C07C\ 249/08$, $C07C\ 249/14$, $C07C\ 251/32$, $C07C\ 251/40$, $C07C\ 251/44$, $B01D\ 61/36$

(21) Anmeldenummer: 02015476.1

(22) Anmeldetag: **12.07.2002**

(54) **Ammoximation von Ketonen und Aufarbeitung durch Pervaporation/Dampfpermeation**

Ammoximation of ketones and method of processing by pervaporation/vapor permeation

Ammoxymation de cétones et procédé de traitement par la pervaporation/perméation de vapeurs

(84) Benannte Vertragsstaaten:
**AT BE BG CH CY CZ DE DK EE ES FI FR GB GR IE IT LI LU MC NL PT SE SK TR**

(30) Priorität: **31.08.2001 DE 10142620**

(43) Veröffentlichungstag der Anmeldung:
**05.03.2003 Patentblatt 2003/10**

(73) Patentinhaber: **Degussa AG**
**40474 Düsseldorf (DE)**

(72) Erfinder:
• **Schiffer, Thomas, Dr.**
**45721 Haltern (DE)**
• **Esser, Peter Ernst, Dr.**
**85609 Aschheim (DE)**
• **Roos, Martin, Dr.**
**45721 Haltern (DE)**
• **Kuppinger, Franz-Felix, Dr.**
**45768 Marl (DE)**
• **Stevermüer, G-nter**
**45770 Marl (DE)**
• **Thiele, Georg Friedrich, Dr.**
**63450 Hanau (DE)**

(56) Entgegenhaltungen:
**EP-A- 0 267 362          EP-A- 0 690 045**

• **LIPNIZKI F ET AL: "Pervaporation-based hybrid process: a review of process design, applications and economics" JOURNAL OF MEMBRANE SCIENCE, ELSEVIER SCIENCE, AMSTERDAM, NL, Bd. 153, Nr. 2, 17. Februar 1999 (1999-02-17), Seiten 183-210, XP004152791 ISSN: 0376-7388**

**Beschreibung**

Die Erfindung betrifft die Aufarbeitung eines Reaktionsgemisches, welches durch Ammoximation eines Ketons mit Wasserstoffperoxid und Ammoniak gebildet wird, wobei der Aufarbeitungsprozess mindestens eine Membrantrennstufe enthält.

In zahlreichen Patentanmeldungen und Patentschriften wird die Ammoximation von Ketonen wie insbesondere Alkanonen und/oder Cycloalkanonen mit Wasserstoffperoxid und Ammoniak an einem heterogenen Katalysatorsystem beschrieben, welches mindestens eine Komponente enthält, die aus den Elementen Titan, Silizium und Sauerstoff aufgebaut ist. Als Beispiele seien hier EP-A-0 299 430, EP-A-0 564 040 und US 5 637 715 genannt.

In der Regel wird als Katalysator ein mikro- oder mesoporöser Titanzeolith verwendet, wobei sich der Titansilikalit TS1 besonders gut für die Ammoximation eignet. Bei großen und sterisch anspruchsvollen Ketonen wie Alkanonen oder Cycloalkanonen ist es außerdem vorteilhaft, das Katalysatorsystem durch weitere Komponenten zu ergänzen. So werden in DE 195 21 011 amorphe Silikate, in DE 100 47 435 saure Feststoffe und in DE 101 03 581 Ammoniumionen als Cokatalysator beschrieben und beansprucht.

Wie in DE 100 47 435 und DE 101 03 581 beschrieben, verläuft die Reaktion bei großen und sterisch anspruchsvollen (Cycle-)Alkanonen wie Cyclododecanon besonders schnell und selektiv in polaren, mit Wasser ganz oder teilweise mischbaren organischen Lösemitteln, insbesondere in kurzkettigen Alkoholen mit 1 bis 6 Kohlenstoffatomen.

Die Ammoximation erfolgt in zwei Teilschritten umfassend die Hydroxylaminbildung (1) und die Oximierung (2). Wasser wird dabei zum einen durch eine wässrige Wasserstoffperoxidlösung eingetragen, zum anderen bildet sich Wasser in stöchiometrischen Mengen bei den beiden Teilschritten als Reaktionsprodukt. Zusätzlich entsteht Wasser auch bei der unproduktiven Zersetzung von Wasserstoffperoxid und Hydroxylamin, formal dargestellt in den Nebenreaktionen (3) und (4) des folgenden am Beispiel des Cyclododecanons (CDON) formulierten Reaktionsschemas:

$$(1) \quad NH_3 + H_2O_2 \rightarrow H_2O + NH_2OH$$

$$(2) \quad NH_2OH + CDON \rightarrow CDON \cdot Oxim + H_2O$$

$$(3) \quad 2\,NH_2OH + H_2O_2 \rightarrow 4\,H_2O + N_2$$

$$(4) \quad 2\,H_2O_2 \rightarrow 2\,H_2O + O_2$$

Während der Umsetzung steigt folglich der Gehalt an Wasser in der Reaktionsmischung an. Sollen große Alkanone oder Cycloalkanone wie zum Beispiel Cyclododecanon ammoximiert werden, so fällt insbesondere die Löslichkeit des entsprechenden Oxims in der Reaktionsmischung mit steigendem Wassergehalt stark ab.

Ziel der Reaktionsführung insbesondere bei großen Cycloalkanonen ist daher, die Menge an Wasser während der Reaktion möglichst zu begrenzen. Dies gelingt gemäß DE 100 47 435 und DE 101 03 581 zum Beispiel dadurch, dass vorteilhaft Ammoniak als trockenes Gas und Wasserstoffperoxid als möglichst konzentrierte Lösung (üblicherweise >30 Gew.-%) verwendet werden. Auch ist es vorteilhaft, wenn der als Lösemittel eingesetzte Alkohol zu Beginn der Reaktion nicht mehr Wasser enthält, als nach einer Destillation im Azeotrop enthalten ist.

Soll der Alkohol mehrfach im Prozess verwendet werden, muss die bei der Reaktion eingetragene Menge an Wasser bei der Aufarbeitung wieder abgetrennt werden.

In den meisten Patentanmeldungen steht die Synthese des Katalysatorsystems, seine Aktivierung und die Ammoximationsreaktion selber im Mittelpunkt der Untersuchungen. Zur Aufarbeitung findet sich in den oben erwähnten Schriften der allgemeine Hinweis, dass der meist pulverförmige Katalysator, in der Regel ein Titansilikalit, über ein Filter oder eine Drucknutsche abgetrennt wird. Anschließend werden Umsatz und Selektivitäten durch GC-Analyse und der Peroxidverbrauch durch Redoxtitration direkt aus der Reaktionslösung bestimmt. Wird die Reaktionsmischung weiter aufgearbeitet, so werden dafür destillative und/oder extraktive Reinigungsschritte gewählt.

ARCO Chemical Technology beschreibt in den europäischen Patentanmeldungen EP-A-0 690 045 und EP-A-0 735 017 einen mehrstufigen Prozess zur Synthese von Caprolactam, wobei für die Stufe der Ammoximation von Cyclohexanon Wasserstoffperoxid aus der Umsetzung von Isopropanol und Sauerstoff verwendet wird. Für den Prozessschritt der Ammoximation von Cyclohexanon wird allgemein jedes geeignete Aufarbeitungsverfahren beansprucht. Als Möglichkeiten werden in EP-A-0 735 017 die Destillation und die Extraktion genannt, ohne diese beiden Verfahren mit experimentellen Daten oder Beispielen zu konkretisieren.

Eine vollständige destillative Trennung von Lösemittel, Edukt und Produkt nach der Stufe der Ammoximation, wie in US 5 451 701 und EP-A-0 690 045 angedacht, mag bei Cyclohexanon-Oxim möglich sein. Nach Destillation des Lösemittels und Wassers lassen sich Cyclohexanon (Sdp. 155 °C/1013 mbar) und Cyclohexanon-Oxim (Sdp. 206 - 210 °C/1013 mbar) destillativ voneinander trennen. Vorteilhaft erfolgt diese Destillation im Vakuum.

Für die Ammoximation makrocyclischer Ketone wie zum Beispiel Cyclododecanon ist ein rein destillatives Verfahren jedoch nicht mehr geeignet. Die destillative Trennung von Keton und Oxim wird mit zunehmender Ringgröße schwieriger, zudem bewirken die hohen Destillationstemperaturen auch im Hochvakuum erhebliche Anteile an Zersetzung. Cyclododecanon-Oxim zum Beispiel lässt sich nicht mehr ohne Zersetzung destillieren.

Mehrere Veröffentlichungen erwähnen bei der Aufarbeitung die Extraktion. Montedipe beschreibt in EP-A-0 208 311, Beispiel 1, die Umsetzung von Cyclohexanon und Aufarbeitung des Ammoximationsproduktes von Cyclohexanon ohne Alkohol als Lösemittel in einem Dreiphasengemisch (organisch-wässrig-fest) bestehend aus Cyclohexanon als organischer Phase, 32 gew.-%igem wässrigen Ammoniak und 32 gew.-%igem wässrigen Wasserstoffperoxid als wässrige Phase, sowie pulverförmigem Titansilikalit als festem Katalysator. Zur Aufarbeitung und Abtrennung des Katalysators wird die organische Phase in Toluol aufgenommen, die wässrige Phase mehrfach mit Toluol extrahiert und der Katalysator durch Filtration abgetrennt.

In der Patentschrift US 4 794 198 und der europäischen Patentanmeldung EP-A-0 267 362 wird ein organisches Solvens, beispielsweise ein Ether, nach der Ammoximation der abgekühlten Reaktionsmischung zugesetzt, mit dem Cyclohexanon und das entsprechende Oxim extrahiert werden.

Enichem destilliert laut EP-A-0 496 385 zunächst ein ammoniakhaltiges Azeotrop der Lösemittel, tert.-Butanol und Wasser ab. Das Oxim und Alkanon werden anschließend in einem Extraktor mit Toluol aus dem Destillationssumpf heraus gewaschen.

Die oben genannten Aufarbeitungsverfahren zeigen vor allem zwei Nachteile:

Das jeweils eingesetzte Keton und sein korrespondierendes Oxim werden erstens mit zunehmender Molekülgröße bezüglich ihres Extraktionsverhaltens immer ähnlicher und lassen sich mit den verwendeten Extraktionsmitteln zwar gemeinsam aus der Reaktionsmischung entfernen, aber nicht oder nur noch unvollständig voneinander trennen. Ein ketonfreies Oxim lässt sich auf diesem Wege nur bei vollständigem Keton-Umsatz erzielen.

Aus zahlreichen Schriften wie zum Beispiel DE 100 47 435 und DE 101 03 581 ist jedoch bekannt, dass die Reaktivität der Ketone gegenüber der Ammoximation mit wachsender Größe abnimmt. Vollständige Umsätze sind bei großen oder sterisch anspruchsvollen Ketonen nur bei langer Reaktionszeit und mit hohem Peroxidverbrauch (= schlechter Peroxidselektivität) möglich.

Weiterer Nachteil der oben erwähnten Aufarbeitungsverfahren ist, dass die Destillation des Lösemittelgemisches einen hohen Energiebedarf erfordert.

Da bei den Ammoximationsverfahren für große Alkanone und Cycloalkanone bevorzugt leichtsiedende, kurzkettige Alkohole mit vorzugsweise 1 bis 6 Kohlenstoffatomen eingesetzt werden, wird bei der Abtrennung des Reaktionswassers durch Rektifikation oder Destillation die gesamte Menge an alkoholischem Lösemittel über Kopf einer oder mehrerer Kolonnen geführt. Das beinhaltet, dass für die gesamte Menge an Lösemittel die jeweilige Verdampfungsenthalpie aufgebracht werden muss. Bei der anschließenden Kondensation des Lösemittels muss diese Energie an ein Kühlmittel abgegeben werden. Trotz Einsatz von Wärmetauschern erfordern diese Prozesse einen erheblichen Energiebedarf, der die Wirtschaftlichkeit dieser Prozesse stark beeinträchtigt.

Zudem ist die oben beschriebene Lösemitteldestillation bei unvollständigen Keton-Umsätzen und/oder bei unvollständiger Abtrennung des Oxims aus der Reaktionsmischung ungeeignet, da sich diese Verbindungen im Abtriebsteil oder Sumpf der Kolonne anreichern und dort auskristallisieren können.

Es bestand daher die Aufgabe, ein energiesparendes Aufarbeitungsverfahren für die Ammoximation von Ketonen, insbesondere von großen und sterisch anspruchsvollen Alkanonen und Cycloalkanonen mit insbesondere 8 bis 20 Kohlenstoffatomen zu finden, bei dem

- der Katalysator nach der Reaktion abgetrennt,
- das Oxim selektiv aus der Reaktionsmischung isoliert,
- das Reaktionswasser vom Lösemittel energiesparend abgetrennt und
- das verbleibende Lösemittel in den Prozess zurückgeführt wird

und welches sich auch bei unvollständigem Umsatz der Ammoximation durchführen lässt.

Überraschend wurde nun gefunden, dass Ketonoxime, insbesondere viele Alkanon-Oxime und Cycloalkanon-Oxime, eine starke Temperaturabhängigkeit ihrer Löslichkeit in kurzkettigen Alkoholen aufweisen. Durch geeignete Temperaturführung lassen sich somit die Oxime auch bei unvollständigem Keton-Umsatz sehr selektiv aus der Reaktionsmischung abtrennen. Dabei werden bereits in einer einstufigen Kristallisation Oxim-Reinheiten von über 99 Gew.-% erzielt, die ausreichend sind für eine direkte weitere Umsetzung der Oxime ohne zusätzliche Reinigungsschritte, beispielsweise in einer Beckmann-Umlagerung.

Zudem wurde überraschend gefunden, dass sich Wasser durch Pervaporation oder Dampfpermeation über Membrane sehr selektiv und energiesparend aus der verbleibenden Mutterlauge entfernen lässt und dieser Aufarbeitungsschritt auch bei unvollständigem Keton-Umsatz und der daraus gegebenen Anwesenheit von Ketonen möglich ist.

Gegenstand der Erfindung ist daher ein Verfahren zur Aufarbeitung eines Reaktionsgemisches, welches durch Ammoximation eines Ketons wie insbesondere Alkanons oder Cycloalkanons mit insbesondere 8 bis 20 Kohlenstoffatomen mit Wasserstoffperoxid und Ammoniak an einem titanhaltigen Katalysator in homogener Lösung gebildet wird, umfassend die Teilschritte

    Abtrennung des Katalysators,
    selektive Abtrennung des bei der Ammoximation gebildeten Oxims,
    Ausschleusung von Reaktionswasser und
    Rückführung des Lösemittels,

dergestalt, dass der Aufarbeitungsprozess auch bei nicht vollständigem Keton-Umsatz durch Verwendung mindestens einer Membrantrennstufe erfolgt.

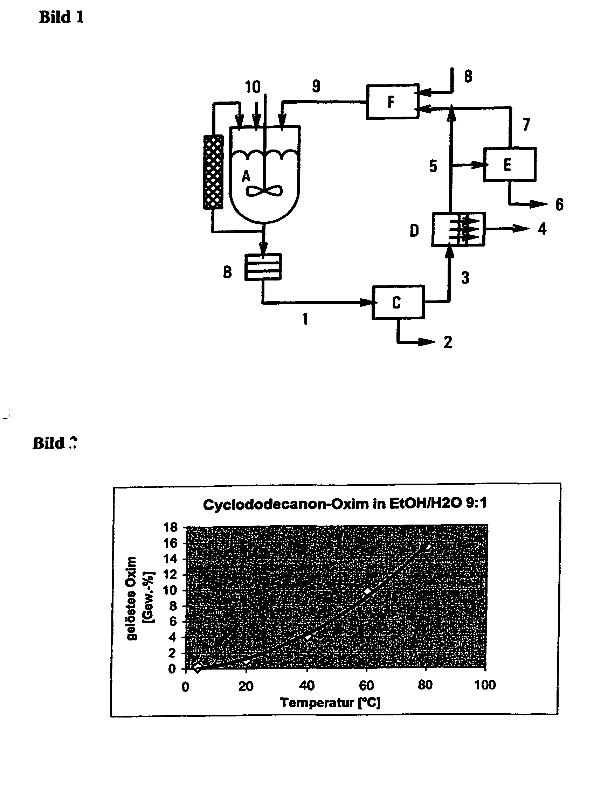

Das erfindungsgemäße Aufarbeitungsverfahren ist in Bild 1 skizziert.

Es kann sowohl kontinuierlich als auch diskontinuierlich gefahren werden.

Der Reaktoraustrag 1 besteht aus einer Lösung eines Keton-Oxims in einem polaren, mit Wasser teilweise oder vollständig mischbaren organischen Lösemittel. Bevorzugt werden als Lösemittel kurzkettige, mit Wasser ganz oder teilweise mischbare Alkohole mit 1 - 6 Kohlenstoffatomen wie zum Beispiel Methanol, Ethanol, n-Propanol, Isopropanol, tert.-Butanol und Amylalkohol verwendet. Wird ein pulverförmiger Katalysator zur Ammoximation verwendet, so kann dieser über ein Filter B abgetrennt werden. Als Filter können alle bekannten Filtertypen wie beispielsweise eine Drucknutsche oder eine Filterzentrifuge verwendet werden.

Wird, wie in Bild 1 skizziert, ein Umlaufreaktor mit Festbett verwendet, so dient Filter B lediglich zur Sicherheit, um feste Verunreinigungen wie zum Beispiel Schwebeteilchen oder Abrieb des Formkörpers aus der Reaktionsmischung zu entfernen.

Die Ammoximation des Ketons im Reaktor A kann sowohl vollständig als auch nur teilweise erfolgen, wobei der Reaktor A in der Zeichnung sowohl einen einstufigen Reaktor als auch ein Verfahren mit mehreren Reaktoren, die in Reihe oder parallel miteinander verschaltet sind, beschreiben soll.

Typischerweise werden bei der Ammoximation Ketonumsätze zwischen 30 % und 100 %, bevorzugt über 50 % erzielt, so dass im Reaktoraustrag neben dem jeweiligen Oxim auch Anteile nicht umgesetzten Eduktes vorliegen können.

Die Reaktionsbedingungen werden so gewählt, dass im Reaktoraustrag (Strom 1) bei den gewählten Bedingungen (Temperatur, Menge Lösemittel und Wassergehalt) Edukt und Produkt vollständig gelöst vorliegen.

Typische Bedingungen für den Reaktoraustrag bei zum Beispiel Cyclododecanon-Oxim sind Temperaturen von 60 °C- 90 °C und Oximkonzentrationen zwischen 5 Gew.-% und 25 Gew.%, wobei die Reaktion vorteilhaft nahe unterhalb der Löslichkeitsgrenze des Oxims gefahren wird, um die Lösemittelmenge zu begrenzen. Der Wasseranteil im Reaktoraustrag beträgt typischerweise 5 Gew.-% - 15 Gew.-%, kann aber auch höher liegen.

Ein weiterer Vorteil des erfindungsgemäßen Aufarbeitungsverfahrens ist, dass optional gemäß DE 101 03 581 in der Reaktionsmischung 1 auch Ammoniumionen als Cokatalysator homogen gelöst sein können. Ihre Konzentration hängt von der Löslichkeit der jeweiligen Salze im Solvens ab. Wird in wässrigen Lösungen kurzkettiger Alkohole wie zum Beispiel Ethanol gearbeitet, beträgt die Ammoniumionenkonzentration typischerweise 0,01 Mol/L - 0,5 Mol/L, wobei als Ammoniumsalze insbesondere diejenigen organischer Carbonsäuren geeignet sind wie zum Beispiel Ammoniumacetat.

Kernstück des erfindungsgemäßen Aufarbeitungskonzeptes sind der Kristaller (Kristallisierbehälter) C in Kombination mit der Pervaporationsstufe/Dampfpermeationsstufe D.

Im Kristaller C erfolgt die Kristallisation des Oxims beim erfindungsgemäßen Verfahren durch Abkühlen des Reaktoraustrags. Optional lässt sich die Kristallisation auch durch Zugabe von weiterem Wasser vervollständigen, wobei zu beachten ist, dass dieses im weiteren Aufarbeitungsschritt D wieder entfernt werden muss.

Der Kristaller wird in der Regel bei Temperaturen zwischen -20 °C und +40 °C, vorteilhaft zwischen -10 °C und +25 °C betrieben. Als Kühlmittel eignen sich Kühlwasser oder Kältesole gut. Die Untergrenze des Temperaturfensters ergibt sich daraus, dass noch kein Wasser im Kristaller ausfriert und dass bei unvollständigem Umsatz die Löslichkeit gegenüber dem Edukt nicht unterschritten wird. In Bild 2 ist exemplarisch die Löslichkeit von Cyclododecanon-Oxim in Abhängigkeit von der Temperatur in einem Gemisch von Ethanol und Wasser im Verhältnis 9 : 1 angegeben.

Das auskristallisierte Oxim (Strom 2) wird als Feststoff abgetrennt. Die mit Mutterlauge benetzten Kristalle können mit wenig Alkohol/Wasser nachgewaschen und getrocknet werden. Ein Umkristallisieren ist prinzipiell möglich, in der Regel jedoch nicht erforderlich. Bei Cyclododecanon-Oxim zum Beispiel wurde in den Versuchen eine Reinheit > 99,9 Gew.-% erzielt. Der Anteil an nicht umgesetztem Cyclododecanon in den Kristallen beträgt in aller Regel < 0,01 Gew.-%. Dies gilt auch für einen unvollständigen Umsatz bei der Ammoximation. Die Kristalle (2) können, in geeigneten Solventien gelöst, direkt zu Folgereaktionen eingesetzt werden. Eine typische Folgereaktion ist die Beckmann-Umlagerung von Cyclododecanon-Oxim zu Laurinlactam in konzentrierter Schwefelsäure.

Die alkoholisch-wässrige Mutterlauge (3) enthält beim erfindungsgemäßen Verfahren noch zwischen 0,01 Gew.-% und 5 Gew.-% Oxim, bevorzugt 0,1 Gew.-% bis 2 Gew.-% Oxim, sowie je nach Umsatz der Ammoximation entsprechende Anteile an Keton. Ferner enthält sie Reste von Ammoniak und Wasserstoffperoxid. Ferner kann sie gemäß DE 101 03 581 optional auch homogen gelöste Ammoniumionen enthalten. Sie wird über einen Wärmetauscher (nicht eingezeichnet) direkt einer Pervaporation oder Dampfpermeation zugeführt. Diese erfolgt typischerweise zwischen 50 °C und 180 °C, vorteilhaft zwischen 80 °C und 140 °C unter dem jeweiligen Dampfdruck des Lösemittels oder einem leichten Überdruck.

Bei der Pervaporation wird die Lösung 3 direkt an der Membran vorbei geleitet, wobei Wasser durch die Membran diffundiert. Werden unvollständige Umsätze mit hohem Keton-Gehalt in Lösung 3 gefahren, kann der Fluss durch die Membran im Laufe der Zeit durch Ablagerung des Ketons auf der Membran verringert werden. Das gleiche Phänomen tritt auch auf, wenn Lösung 3 Ammoniumionen enthält. Überraschend zeigte sich hier wirksame Abhilfe durch eine leichte Modifikation in Form der Dampfpermeation.

Bei der Dampfpermeation bildet sich über der Lösung 3 eine Gasblase. Diese wird in einem Verdichter komprimiert oder wieder verflüssigt und an der Membran vorbeigeleitet, wobei daraus selektiv das Wasser entzogen wird. Hinter der Membran erfolgt die Entspannung über eine Drossel, die Dämpfe werden aufgeheizt und durch die Lösung 3 geleitet. Dabei wird aus der Lösung neues Wasser verdampft. Salze und Keton bleiben in Lösung 3 zurück. Dadurch lässt sich die Verstopfung der Membran minimieren.

Der energetische Vorteil bei beiden Membranverfahren, Pervaporation und der Dampfpermeation, ist, dass nur die Verdampfungsenthalpie des ausgetragenen Wassers aufgebracht werden muss, während bei einer Destillation die Verdampfungsenthalpie der gesamten Menge Lösemittel (Alkohol) aufgebracht werden muss.

Für die Selektivität ist der Membrantyp und sein Porendurchmesser entscheidend. Als Membrane eignen sich sowohl Polymermembrane, beispielsweise auf Basis Polystyrol, Polyacrylat und Polysiloxane, als auch anorganische Membranen beispielsweise mit Zeolith- oder Silica-Strukturen.

Der ausgetragene Teilstrom (4) enthält diejenige Menge an Wasser, die durch den Zulauf (10) an wässriger Wasserstoffperoxidlösung und Ammoniak, sowie dem gebildeten Reaktionswasser im Ammoximationsreaktor A entstanden ist. Wurde im Kristaller C Wasser zugesetzt, muss auch dieses wieder entzogen werden.

Üblicherweise wird in D der Anteil Wasser von 8 Gew.-% -15 Gew.-% im Zulauf (Strom 3) auf circa 5 Gew.-% in Strom 5 reduziert. In diesem Bereich arbeiten die verwendeten Membrane sehr selektiv und zeigen gute Durchflussraten. Jedoch kann der Wassergehalt im Einsatz auch auf unter 1 Gew.-% reduziert werden und damit deutlich unter den Gehalt gebracht werden, der sich beispielsweise bei einer Destillation ohne Schleppmittel als Azeotrop einstellt.

Der Anteil an Alkohol, Ammoniak oder anderen Lösemittelkomponenten, die durch die Membran diffundieren (Strom 4), ist in der Regel gering. Oxim und Keton gelangen nicht oder nur in Spuren durch die Membran. Eine destillative oder andere Nachbehandlung dieser Phase ist in der Regel nicht erforderlich.

Die vom Reaktionswasser weitgehend befreite Phase 5 kann in einem Mischer F mit neuem Keton (8) versetzt und zurück in den Reaktor geführt werden.

Bei einem kontinuierlichen Prozess mit technisch reinen Einsatzstoffen können sich im Laufe der Zeit Nebenprodukte anreichern, die gegenüber der Ammoximation inaktiv sind. So enthält zum Beispiel technisch reines Cyclododecanon meist Spuren von Cyclododecan und Cyclododecanol. Es ist daher vorteilhaft, kontinuierlich oder diskontinuierlich einen Teilstrom von 5 abzutrennen und diesen destillativ von den Nebenkomponenten zu reinigen. Das so gereinigte Lösemittelgemisch 7 wird mit dem Hauptstrom 5 vereinigt in den Mischer F und dann in den Ammoximationsreaktor zurückgeführt.

Die Nebenkomponenten werden in Teilstrom 6 aus dem Prozess ausgeschleust.

Das Verfahren eignet sich insbesondere für die Ammoximation von großen und sterisch anspruchsvollen Alkanonen und Cycloalkanonen mit insbesondere 8 bis 20 Kohlenstoffatomen wie zum Beispiel Cyclooctanon, Cyclodecanon, Cyclododecanon, Cyclopentadecanon und Acetophenon.

Beispiele

**Beispiel 1 (Ammoximation):**

In einem 1,6 L Autoklav werden 73 g (400 mmol) Cyclododecanon (CDON) in 535 g ca. 96%igem Ethanol gelöst. Bei 80 °C wird trockenes Ammoniakgas in den Reaktor geleitet und ein Druck von 1,6 bar eingestellt (ca. 14 g Ammoniak). Die Reaktionsmischung wird mit einer Umwälzpumpe mit 600 mL/min über ein Festbett mit 200 g Katalysatorformkörper (Titansilikalit TS-1 mit 20 Gew.-% saurem Aluminiumoxid gemäß EP 100 47 435, Degussa AG) geleitet. Über eine Reaktionszeit von 4 Stunden werden 40,8 g einer 50 gew.-%igen wässrigen Wasserstoffperoxidlösung (= 600 mmol $H_2O_2$) zudosiert. Die Dosierung erfolgt vor dem Reaktor. Gasförmige Nebenprodukte werden über eine Drossel aus der Reaktionsmischung entfernt, mit ausgetragener Ammoniak wird nachdosiert, ca. 2 g innerhalb von 240 Minuten. Nach beendeter Zugabe wird die Reaktionsmischung noch 60 Minuten nachgerührt. Der CDON-Umsatz zum Oxim beträgt 95,3 % (GC-Analyse). Der Anteil an Nebenprodukten liegt unter 0,1 %.

**Beispiel 2 (Ammoximation):**

Der Ansatz aus Beispiel 1 wird wiederholt, es werden zusätzlich 5,8 g (0,1 mol/L) Ammoniumacetat gemäß EP 101 03 581 homogen in der Reaktionsmischung gelöst. Es werden 523 mmol $H_2O_2$ innerhalb von 180 Minuten zudosiert, die Reaktionsmischung anschließend noch 60 Minuten nachgerührt. Der CDON-Umsatz beträgt > 99,9% (GC-Analyse).

**Beispiel 3 (Kristallisation):**

Die Reaktionsmischung aus Beispiel 1 wird aus dem Autoklav ausgefahren, nicht umgesetztes Ammoniakgas entweicht beim Entspannen und kann über eine Kühlfalle kondensiert und in den Prozess zurückgeführt werden. Die Reaktionsmischung wird auf 4 °C abgekühlt und der ausgefallene Niederschlag nach 2 Stunden über eine Nutsche abgetrennt. Der Niederschlag wird mit wenig kaltem Ethanol / Wasser 9 : 1 nachgewaschen. Nach dem Trocknen bei 60 °C / 200 mbar werden 72,6 g CDON-Oxim in einer Reinheit >99,8% gewonnen.

Die Mutterlauge (ca. 590 g) besteht aus wässrigem Ethanol, in dem noch ca. 3,4 g CDON und ca. 2,6 g Oxim gelöst vorliegen. Durch eingetragenes und während der Ammoximation frei gewordenes Wasser, sowie der Waschlösung nach der Krstallisation steigt der Wassergehalt in der ethanolischen Lösung von ca. 4 Gew.-% auf ca. 11 Gew.-% (ca. 62 g) an.

**Beispiel 4 (Kristallisation):**

Analog zu Beispiel 3 wird die Reaktionsmischung aus Beispiel 2 aufgearbeitet. Es werden 76,4 g CDON-Oxim mit einer Reinheit > 99,8 % gewonnen. Die Mutterlauge (ca. 580 g) besteht aus wässrigem Ethanol, in dem noch ca. 2,5 g Oxim und < 0,1 g CDON gelöst vorliegen. Der Wassergehalt liegt bei ca. 10 Gew.-%. (ca. 58 g). In der Mutterlauge sind 5,8 g Ammoniumacetat gelöst.

**Beispiel 5 (Pervaporation):**

Eine Probe der Mutterlauge aus Beispiel 3 wird bei 90 °C an einer Polymermembran, Typ Sulzer Chemtech 2201 vorbeigeleitet, bis der Wassergehalt in der Lösung auf ca. 4 % gesunken ist. Der Wassergehalt im Permeat liegt über 98 %, der Fluss fällt während des Versuchs mit sinkendem Wassergehalt im Eduktzufuhrstrom (Feed) von 0,5 kg/m$^2$*h auf 0,2 kg/m$^2$*h ab.

**Beispiel 6 (Pervaporation):**

Eine Probe der Mutterlauge aus Beispiel 3 wird analog zu Beispiel 5 bei 120 °C an einer NaA-Zeolithmembran (Firma Mitsui) vorbeigeleitet. Der Wassergehalt im Permeat liegt bei 96 % und sinkt zum Ende des Versuchs auf 94 % ab. Der Fluss fällt während des Versuchs mit sinkendem Wassergehalt im Eduktzufuhrstrom (Feed) von 3,5 kg/m$^2$*h auf 2,6 kg/m$^2$*h ab.

**Beispiel 7 (Pervaporation):**

Der Versuch aus Beispiel 6 wird mit einer NaY-Membran (Firma Mitsui) bei 80 °C wiederholt. Der Wassergehalt im Permeat liegt bei 78 % und sinkt zum Ende des Versuchs auf 68 % ab. Der Fluss fällt während des Versuchs mit sinkendem Wassergehalt im Eduktzufuhrstrom (Feed) von 2,8 kg/m$^2$*h auf 1,7 kg/m$^2$*h ab.

**Beispiel 8 (Pervaporation)**

Der Versuch aus Beispiel 6 wird mit einer anorganischen Silica-Membran (Sulzer Chemtech SMS) bei 140 °C wiederholt. Der Wassergehalt im Permeat liegt bei 88 % und sinkt zum Ende des Versuchs auf 70 % ab. Der Fluss fällt mit sinkendem Wassergehalt im Eduktzufuhrstrom (Feed) von 10,1 kg/(m$^2$*h) auf 5,0 kg/(m$^2$*h) ab.

**Beispiel 9 (Dampfpermeation):**

Eine ammoniumacetathaltige Probe aus Beispiel 4 wird partiell verdampft, der Dampf über einen Tröpfchenfänger geleitet, komprimiert und an einer anorganischen Silicamembran (Sulzer Chemtech SMS) vorbeigeleitet. Der Wassergehalt im Permeat beträgt 90 % - 92 %, der Fluss liegt bei 0,8 kg/m$^2$*h.

**Patentansprüche**

1. Verfahren zur Aufarbeitung eines Reaktionsgemisches, welches durch Ammoximation eines Ketons mit Wasserstoffperoxid und Ammoniak an einem titanhaltigen Katalysator in homogener Lösung gebildet wird, mit den Teilschritten
Abtrennung des Katalysators,
selektive Abtrennung des bei der Ammoximation gebildeten Oxims,
Ausschleusung von Reaktionswasser und Rückführung des Lösemittels,
**dadurch gekennzeichnet,**
**dass** zumindest ein Teil des in der Mutterlauge enthaltenen Wassers in einer Membrantrennstufe abgetrennt und aus dem Reaktionskreislauf ausgeschleust wird.

2. Verfahren nach Anspruch 1,
**dadurch gekennzeichnet,**
**dass** als Lösemittel der Ammoximation eine mit Wasser gut oder vollständig mischbare polare, organische Flüssigkeit verwendet wird.

3. Verfahren nach einem der vorhergehenden Ansprüche,
**dadurch gekennzeichnet,**
**dass** als Lösemittel der Ammoximation ein kurzkettiger Alkohol aus der Gruppe, die durch Methanol, Ethanol, n-Propanol, Isopropanol, tert.-Butanol und Amylalkohol gebildet wird, ausgewählt wird.

4. Verfahren nach einem der vorhergehenden Ansprüche,
**dadurch gekennzeichnet,**

**dass** das Produkt durch eine Kristallisation selektiv aus dem Prozess abgetrennt wird und das Wasser aus der Mutterlauge durch Pervaporation abgetrennt wird.

5. Verfahren nach einem der Ansprüche 1 bis 3,
   **dadurch gekennzeichnet,**
   **dass** das Produkt zunächst durch eine Kristallisation aus dem Prozess abgetrennt wird und das Wasser aus der Mutterlauge durch Dampfpermeation abgetrennt wird.

6. Verfahren nach einem der vorgehenden Ansprüche,
   **dadurch gekennzeichnet,**
   **dass** der Anteil an Wasser in der Mutterlauge mittles einer Membrantrennstufe auf etwa 5 Gew. % reduziert wird.

7. Verfahren nach einem der Ansprüche 1 bis 5,
   **dadurch gekennzeichnet,**
   **dass** der Anteil an Wasser in der Mutterlauge mittles einer Membrantrennstufe auf unter 5 Gew. %, insbesondere unter 1 Gew. % reduziert wird.

8. Verfahren nach einem der vorgehenden Ansprüche,
   **dadurch gekennzeichnet,**
   **dass** das polare organische Lösemittel aus der Mutterlauge in den Ammoximationsreaktor zurückgefahren wird.

9. Verfahren nach einem der vorhergehenden Ansprüche,
   **dadurch gekennzeichnet,**
   **dass** als Membran eine Polymermembran verwendet wird.

10. Verfahren nach Anspruch 9,
    **dadurch gekennzeichnet,**
    **dass** die Polymermembran aus der Gruppe, die durch Polystyrol, Polyacrylate und Polysiloxane gebildet wird, ausgewählt wird.

11. Verfahren nach einem der Ansprüche 1 bis 8,
    **dadurch gekennzeichnet,**
    **dass** die Membran aus anorganischem Material besteht.

12. Verfahren nach Anspruch 11,
    **dadurch gekennzeichnet,**
    **dass** die Membran eine Zeolith- oder Silica-Struktur aufweist.

13. Verfahren nach einem der vorgehenden Ansprüchen,
    **dadurch gekennzeichnet,**
    **dass** der Kristaller bei Temperaturen zwischen -20 °C und +40 °C betrieben wird.

14. Verfahren nach einem der vorgehenden Ansprüche,
    **dadurch gekennzeichnet,**
    **dass** die Pervaporation oder Dampfpermeation bei 50 °C bis 180°C erfolgt.

15. Verfahren nach einem der vorgehenden Ansprüche,
    **dadurch gekennzeichnet,**
    **dass** die Pervaporation oder Dampfpermeation bei 80 °C bis 140 °C erfolgt

16. Verfahren nach einem der vorhergehenden Ansprüche,
    **dadurch gekennzeichnet,**
    **dass** das Keton aus der Gruppe, die durch Cyclooctanon, Cyclodecanon, Cyclododecanon, Cyclopentadecanon und Acetophenon gebildet wird, ausgewählt wird.

**Claims**

1. A process for the work-up of a reaction mixture formed by ammoximation of a ketone by means of hydrogen peroxide and ammonia in homogeneous solution over a titanium-containing catalyst, which comprises the substeps
   removal of the catalyst,
   selective separation of the oxime formed in the ammoximation from the reaction mixture,
   removal of water of reaction and
   recirculation of the solvent,
   wherein at least part of the water contained in the mother liquor is removed of the reaction mixture by membrane separation.

2. The process as claimed in claim 1, wherein a polar, organic liquid which is substantially or completely miscible with water is used as solvent in the ammoximation.

3. The process as claimed in either of the preceding claims, wherein the solvent used in the ammoximation is a short-chain alcohol selected from the group consisting of methanol, ethanol, n-propanol, isopropanol, tert-butanol and amyl alcohol.

4. The process as claimed in any of the preceding claims, wherein the product is selectively separated off from the process by crystallization and the water of reaction is separated off from the mother liquor by pervaporation.

5. The process as claimed in any of claims 1 to 3, wherein the product is firstly separated off from the process by crystallization and the water of reaction is separated off from the mother liquor by vapor permeation.

6. The process as claimed in any of the preceding claims, wherein the proportion of water in the mother liquor is reduced to about 5 % by weight by membrane separation.

7. The process as claimed in any of claims 1 to 5, wherein the proportion of water in the mother liquor is reduced below 5 % by weight in particular below 1 % by weight by membrane separation.

8. The process as claimed in any of the preceding claims, wherein the polar organic solvent from the mother liquor is returned to the ammoximation reactor.

9. The process as claimed in any of the preceding claims, wherein the membrane used is a polymer membrane.

10. The process as claimed in claim 9, wherein the polymer membrane is selected from the group consisting of polystyrene, polyacrylates and polysiloxanes.

11. The process as claimed in any of claims 1 to 8, wherein the membrane comprises inorganic material.

12. The process as claimed in claim 11, wherein the membrane has a zeolite or silica structure.

13. The process as claimed in any of the preceding claims, wherein the crystallizer is operated at temperatures in the range from -20°C to +40°C.

14. The process as claimed in any of the preceding claims, wherein the pervaporation or vapor permeation is carried out at from 50°C to 180°C.

15. The process as claimed in any of the preceding claims, wherein the pervaporation or vapor permeation is carried out at from 80°C to 140°C.

16. The process as claimed in any of the preceding claims, wherein the ketone is selected from the group consisting of cyclooctanone, cyclodecanone, cyclododecanone, cyclopentadecanone and acetophenone.

**Revendications**

1. Procédé de traitement d'un mélange réactionnel formé par ammoximation d'une cétone avec du peroxyde d'hydrogène et de l'ammoniac sur un catalyseur contenant du titane en solution homogène, avec les étapes suivantes :

- séparation du catalyseur,
- séparation sélective de l'oxime formée lors de l'ammoximation,
- éclusage de l'eau de réaction et
- recirculation du solvant,

**caractérisé en ce qu'**
au moins une partie de l'eau contenue dans la lessive mère est séparée dans une étape de séparation à membrane et éclusée du circuit réactionnel.

2. Procédé selon la revendication 1,
**caractérisé en ce que**
comme solvant de l'ammoximation, on utilise un liquide organique polaire bien ou entièrement miscible à l'eau.

3. Procédé selon l'une quelconque des revendications précédentes,
**caractérisé en ce que**
comme solvant de l'ammoximation, on choisit un alcool à chaîne courte dans le groupe constitué de méthanol, d'éthanol, de n-propanol, d'isopropanol, de tert-butanol et d'alcool amylique.

4. Procédé selon l'une quelconque des revendications précédentes,
**caractérisé en ce que**
le produit est séparé du processus par cristallisation sélective et l'eau est séparée de la lessive mère par pervaporation.

5. Procédé selon l'une quelconque des revendications 1 à 3,
**caractérisé en ce que**
le produit est séparé du processus d'abord par cristallisation puis l'eau est séparée de la lessive mère par perméation de vapeur.

6. Procédé selon l'une quelconque des revendications précédentes,
**caractérisé en ce que**
la fraction d'eau dans la lessive mère est réduite à environ 5 % en poids au moyen d'une étape de séparation à membrane.

7. Procédé selon l'une quelconque des revendications 1 à 5,
**caractérisé en ce que**
la fraction d'eau dans la lessive mère est réduite à moins de 5 % en poids, en particulier à moins de 1 % en poids, au moyen d'une étape de séparation à membrane.

8. Procédé selon l'une quelconque des revendications précédentes,
**caractérisé en ce que**
le solvant organique polaire est recyclé depuis la lessive mère dans le réacteur d'ammoximation.

9.  Procédé selon l'une quelconque des revendications précédentes,
    **caractérisé en ce que**
    la membrane utilisée est une membrane polymère.

10. Procédé selon la revendication 9,
    **caractérisé en ce que**
    la membrane polymère est choisie dans le groupe constitué de polystyrène, de polyacrylates et de polysiloxanes.

11. Procédé selon l'une quelconque des revendications 1 à 8,
    **caractérisé en ce que**
    la membrane est en matériau inorganique.

12. Procédé selon la revendication 11,
    **caractérisé en ce que**
    la membrane présente une structure de zéolithe ou de silice.

13. Procédé selon l'une quelconque des revendications précédentes,
    **caractérisé en ce que**
    le cristalliseur fonctionne à des températures comprises entre -20°C et +40°C.

14. Procédé selon l'une quelconque des revendications précédentes,
    **caractérisé en ce que**
    la pervaporation, ou la perméation de vapeur, est réalisée à une température de 50°C à 180°C.

15. Procédé selon l'une quelconque des revendications précédentes,
    **caractérisé en ce que**
    la pervaporation, ou la perméation de vapeur, est réalisée à une température de 80°C à 140°C.

16. Procédé selon l'une quelconque des revendications précédentes,
    **caractérisé en ce que**
    la cétone est choisie dans le groupe constitué de cyclooctanone, cyclodécanone, cyclododécanone, cyclopentadécanone et acétophénone.

**Bild 1**

**Bild 2**

Cyclododecanon-Oxim in EtOH/H2O 9:1